# EUROPEAN PATENT SPECIFICATION

(11) **EP 1 695 658 B1**
(45) Date of publication and mention of the grant of the patent: **02.01.2008**
(21) Application number: 05251177.1
(22) Date of filing: 28.02.2005
(51) Int. Cl.: A61B 5/00, A61B 19/02, G01N 25/72, G01J 5/04

(54) **Mobile thermal texture mapping apparatus**
Mobile Vorrichtung zur thermalen Texturabbildung
Appareil mobile pour le mappage de texture thermique

(43) Date of publication of application: 30.08.2006
(73) Proprietor: Liu, Zhongqi, Beijing 100088 (CN)
(72) Inventor: Liu, Zhongqi, Beijing 100088 (CN)
(74) Representative: Rees, Alexander Ellison

(56) References cited:
- DE-A1- 3 532 370
- US-A- 5 637 871
- US-A- 6 023 637
- US-A1- 2004 159 790

## Description

The present invention relates to a mobile thermal texture mapping (TTM) or thermal imaging apparatus which can be used to perform thermal texture mapping (TTM) or thermal imaging evaluation on a patient in the field, in remote areas, in emergency situations or in mobile situations leading to clinical diagnosis.

Since the early 21^{st} century, TTM technology has attracted wide attention from within the scientific and medical fields. In particular, in the medical field TTM technology can be used to enable the operator to analyse and determine the source of thermal radiation within a patient's body by obtaining infrared thermal radiation data at the surface of the patient's body. It has been found from a number of statistical studies using TTM technology that the source of thermal radiation within a patient's body can be indicative of a number of illnesses, diseases or conditions. Furthermore, it has been found that relationships exist between the illness, disease or condition and the source of the thermal radiation within a patient's body. The TTM technique is discussed further in United States Patent no. 6,023,637 and the relevant contents of which are hereby incorporated by reference.

The prior art TTM apparatus usually consists of an operation and central processing system, a scanning system and a scanning bed. The operation and central processing system includes a console, a central processing unit, a color displayer and a color printer. The operator uses the console to control the scanning system and scanning bed. The scanning system comprises three parts: a scanning head, a scanning shelf and a scanning cover. The scanning shelf is usually present inside the scanning cover. Alternatively, the scanning shelf and scanning cover may form a single component and the scanning head is supported above the scanning shelf on a moveable floating table. The operator uses the console to move the scanning head. The scanning head can be raised or lowered or moved left, right, up or down in relation to the patient. The console can also be used to focus the scanning head. The scanning bed can also be moved forward or backward along rails. A rotary portion is provided at the lower portion of the bed so as to enable 360° rotation of the bed to meet the scanning requirements.

The prior art TTM apparatus as discussed above requires a large amount of space and also demands a high power supply. The TTM apparatus of the prior art is therefore difficult to move or relocate. The prior art TTM apparatus is therefore not suitable for use in the evaluation leading to the clinical diagnosis of patients in the field, in remote areas, in emergency situations such as the army or in mobile situations such as evaluating passengers in vehicles or on board ships.

US-6023637 discloses a thermal imaging system comprising an infrared scanning system. The infrared scanning system includes an infrared sensor and associated optics for scanning an anatomical area of interest. The system generates electrical signals which vary in intensity according to the intensity of thermal radiation at each point in the scanned area. After digitization, the thermal energy signals are mapped to a color spectrum according to the intensity of the thermal energy at each point in the scanned image.

US-5637871 discloses a highly portable infrared thermography system. The system includes a portable infrared camera, a digital data processing and recording device, a digital display means, means for selecting individual frames and digital storing means.

Accordingly, the present invention provides a mobile TTM apparatus comprising:
a) an infrared scanning head,
b) a direction-controlling floating table,
c) a scanning head direction-controller,
d) a central processing unit,
e) a power unit comprising a self-contained power supply and/or an external power supply interface, and
f) a container comprising an outer container and at least one inner container,
in which when the apparatus is not in use the infrared scanning head, the direction-controlling floating table, the scanning head direction-controller, the central processing unit and the power unit are stored within the container;
and in which when the apparatus is in use the infrared scanning head and the direction-controlling floating table are removed from the container and the infrared scanning head is located on the direction-controlling floating table which is supported by at least one surface of the container;
characterized in that the inner and outer containers of the apparatus further comprise sliding rails and guiding slots to enable vertical displacement of the inner containers relative to further inner containers and the outer container, and said inner containers are secured in position by fixation means.

In a one embodiment, when the apparatus is in use the infrared scanning head is connected to the scanning head direction-controller, the central processing unit and the power unit by signal lines,

In a further embodiment, when the apparatus is in use preferably one or more of the scanning-head direction controller, the central processing unit and the power unit are additionally removed from the container.

In a further embodiment, when the apparatus is in use the power supply is stored within the container.

Preferably, the apparatus comprises an outer container and at least one inner container. Preferably the apparatus comprises one inner container. More preferably the apparatus comprises two inner containers. Preferably the at least one inner container is capable of being inserted into the outer container. The outer container preferably provides at least one opening through which the at least one inner container can be inserted. The at least one inner container preferably provides at least one opening through which a further inner container can be inserted. The inner containers are preferably capable of being inserted within each other and within the outer container so as to form a single container.

Preferably, when not in use the infrared scanning head, a direction-controlling floating table, a scanning head direction-controller, a central processing unit and a power unit are stored within the at least one inner container within the outer container of the present invention.

In one embodiment of the invention, the apparatus of the present invention comprises an outer container and at least one inner container in which at least one inner container can be displaced in one direction with respect to the outer container. Preferably, the at least one inner container is partially withdrawn from the outer container through the opening of the outer container. Preferably, a plurality of inner containers are partially withdrawn from the outer container. Each inner container is preferably partially withdrawn from a further inner container. The inner containers are preferably partially withdrawn from the outer container and/or additional inner containers and secured in place.

Preferably, the at least one inner container is partially withdrawn from the outer container resulting in the outer surfaces of at least one of the inner containers being exposed. Preferably, the at least one inner container is displaced in one direction relative to the outer container by a pre-determined distance. Preferably the at least one inner container is displaced vertically relative to the outer container. Preferably, at least one of the exposed outer surfaces of the at least one inner container provides a stable, supporting surface for the direction-controlling floating table of the TTM apparatus. Preferably, the stable, supporting surface is provided by the upper exposed surface of one of the exposed inner containers protruding from the outer container of the TTM apparatus.

Preferably, the at least one inner container comprises sliding rails and guiding slots enabling the inner container to be displaced vertically relative to further inner containers and/or the outer container of the present invention. Preferably, the outer container additionally comprises sliding rails and guiding slots enabling the at least one inner container to be displaced vertically relative to the outer container. Preferably, the inner containers are secured in position relative to each other and/or relative to the outer container by fixation means. The fixation means include, for example, locating pins or blocks.

Preferably, one or more inner containers are withdrawn partially from the outer container and secured in place so that at least one exposed surface of the inner containers provides the stable, supporting surface for the direction-controlling floating table of the TTM apparatus at the required height. Displacing at least one inner container vertically relative to further inner containers and/or the outer container provides a height-adjustable supporting surface for the direction-controlling floating table of the TTM apparatus. The scanning head is located on the direction-controlling floating table and therefore the present invention provides a method for adjusting the height of the scanning head.

The present invention therefore has the advantage that when not in use the apparatus is stored in a compact manner within the outer container. The present invention therefore stores the apparatus within a small volume of space. In use, the infrared scanning head and direction-controlling floating table and optionally one or more of a scanning head direction-controller, a central processing unit and a power unit are removed from the container. The direction-controlling floating table is supported by at least one surface of the container. Advantageously, the height of the supporting surface provided by at least one of the inner containers relative to the outer container can be adjusted in accordance with the necessary operating conditions without unnecessary extra movement of the patient.

In a further embodiment, the outer surface of the container additionally comprises at least one handle which enables the TTM apparatus of the present invention to be transported. Preferably, the outer container additionally comprises wheels which further enable the TTM apparatus of the present invention to be transported.

The present invention therefore has the advantage that the apparatus is mobile and therefore can be used in the field, in remote areas such a for field workers, in emergency situations such as in the army and in mobile situations such as in vehicles and on passenger ships.

In a further embodiment, the apparatus of the present invention is suitable for use with a 12V DC power supply. The power supply can be provided, for example by an external 12V DC supply provided by an automobile engine.

Preferably, the power supply suitable for use with the apparatus of the present invention is capable of supplying power for a sufficient amount of time to enable the analysis of the patient to be carried out. The power supply for use with the apparatus of the present invention is therefore capable of supplying the necessary power for at least about 40 minutes, preferably at least about 60 minutes. For example, the power supply is capable of supplying the power for between 40 and 60 minutes.

In a further embodiment, the apparatus of the present invention preferably includes a self-contained accumulator and an interface for connecting the apparatus of the present invention with an external 12V DC power supply, from for example an automobile engine.

In a further embodiment, the apparatus of the present invention is suitable for use with an AC power supply. Preferably, the apparatus of the present invention further comprises a transformer to transfer an AC power supply into a 12V DC power supply. Preferably, the transformer is capable of transferring the commercial AC power supply of for example 220V or 110V to a 12V DC power supply.

In a further embodiment, the apparatus of the present invention further comprises a man-powered generator. The apparatus of the present invention therefore has the advantage that the apparatus can be used in areas where there is no access to either a DC or AC power supply. Preferably the man-powered generator is capable of supplying a 12V DC power supply. The present invention has the advantage that the apparatus can be used in remote areas where there is no access to a power supply.

The apparatus of the present invention can be adapted to the operating conditions to include one or more of the transformer and the man-powered generator. The present invention therefore has the advantage that the apparatus can be adapted so as to be used over a wide range of operating conditions.
Embodiments of the invention will now be described in a non-limitative sense by way of example with reference to the accompanying drawings, in which:
Fig. 1 is an illustration of the mobile TTM apparatus of the present invention in use;
Fig. 2 is a diagram illustrating the operation processes for the mobile TTM apparatus of the present invention;

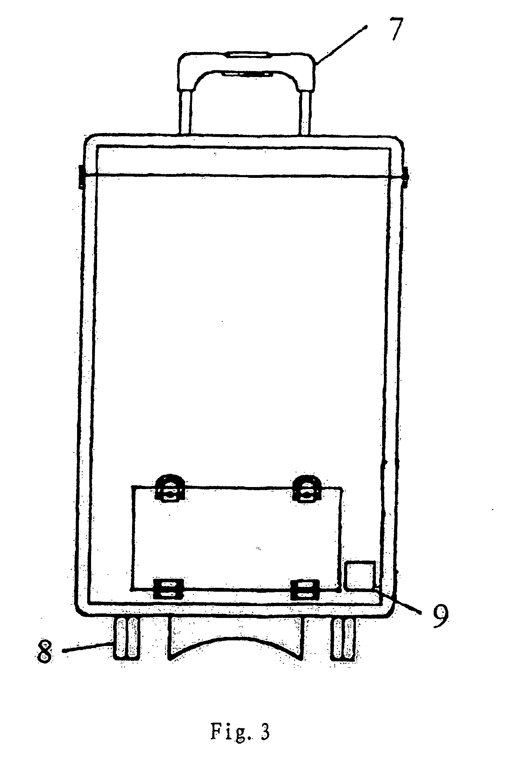

Fig. 3 is the rear view of the outer container when the TTM apparatus is not in use.

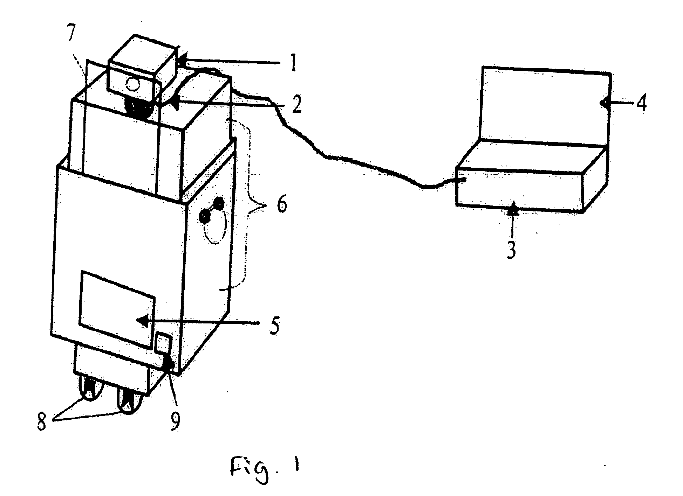

Fig. 1 illustrates the apparatus of the present invention in use. The apparatus comprises an infrared scanning head 1, a direction-controlling floatable table 2, a scanning head direction-controller 3, a central processing unit 4, a self-contained power unit 5, a container 6 and an external power interface 9.
When the apparatus is not in use the infrared scanning head 1, a direction-controlling floatable table 2, a scanning head direction-controller 3, a central processing unit 4, a self-contained power unit 5 and an external power interface 9 are stored within the container 6.
In use the scanning head 1 is removed from the container 6 and the direction-controlling floating table 2 is placed on the stable supporting surface provided by the upper exposed surface of the inner container. The scanning head 1 respectively connects with the central processing unit 4 and the scanning head direction-controller 3 through signal lines. The central processing unit 4 and the scanning-direction controller 3 can be removed from the container 6 for the operator to perform scanning operations and for processing data. The power unit 5 or power from an external power interface 9 for connection to an external power source provides power to the apparatus.
The apparatus comprises an outer container and at least one inner container. The power unit is placed inside the container. The apparatus comprises sliding rails and guiding slots which enable the inner containers to move vertically in relation to the outer container. The upper surface of the inner container provides a stable supporting surface for the direction-controlling floatable table 2 for supporting the scanning head 1. The vertical displacement of the scanning head 1 can be adjusted by adjusting the vertical displacement of the inner containers in relation to each other and in relation to the outer container. The inner containers can be secured in position relative to each other and relative to the outer container using locating blocks.
The apparatus includes a handle 7 which is located on one of the outer surfaces of the outer container. The handle can be elongated or shortened for convenient movement of the outer container. The apparatus further includes wheels 8. The wheels 8 are located on the lower outer surface of the outer container.
Depending on the circumstances in which the apparatus of the present invention is to be used the apparatus may further include a transformer and/or a man-powered generator.

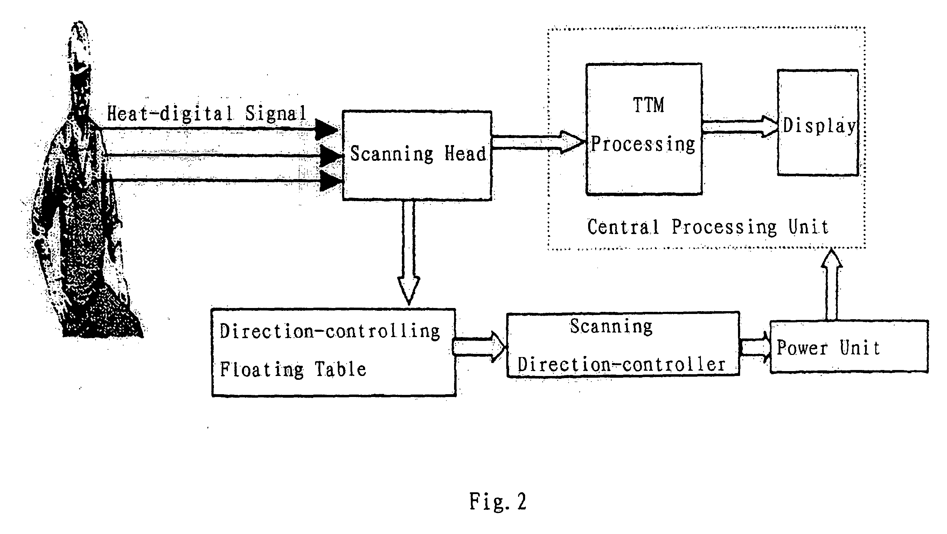

Fig. 2 illustrates the operation processes for the mobile TTM apparatus of the present invention. The floating table controller gives an instruction to the direction-controlling floating table. The scanning direction is adjusted by the direction-controlling floating table to the required scanning direction. The heat radiated during the metabolism course of human cells is detected by the scanning head and is transferred into digital signals, then transmitted to the central processing unit. After TTM's processing, a thermal radiation distribution map, reflecting the relative metabolism intensity of the detected parts of the patient's body is displayed and further analysis of the patient can be performed to evaluate the condition of the patient.

## Claims

1. A mobile thermal texture mapping apparatus comprising:
a) an infrared scanning head (1),
b) a direction-controlling floating table (2),
c) a scanning head direction-controller (3),
d) a central processing unit (4),
e) a power unit comprising a self-contained power supply (5) and/or an external power supply interface (9), and
f) a container (6) comprising an outer container and at least one inner container,
in which when the apparatus is not in use the infrared scanning head (1), the direction-controlling floating table (2), the scanning head direction-controller (3), the central processing unit (4) and the power unit are stored within the container (6);
and in which when the apparatus is in use the infrared scanning head (1) and the direction-controlling floating table (2) are removed from the container (6) and the infrared scanning head (1) is located on the direction-controlling floating table (2) which is supported by at least one surface of the container (6);
**characterized in that** the inner and outer containers of the apparatus further comprise sliding rails and guiding slots to enable vertical displacement of the inner containers relative to further inner containers and the outer container, and said inner containers are secured in position by fixation means.

2. The mobile thermal texture mapping apparatus as claimed in claim 1 in which the at least one inner container can be displaced vertically relative to the outer container and secured at a required vertical displacement to provide a stable, supporting surface for the direction-controlling floating table (2).

3. The mobile thermal texture mapping apparatus as claimed in claim 2 in which the container (6) comprises an outer container and two inner containers.

4. The mobile thermal texture mapping apparatus as claimed in claim 1 in which the fixation means are selected from locating pins or blocks.

5. The mobile thermal texture mapping apparatus as claimed in any one of claims 1 to 4 in which at least outer surface of the container (6) further comprises at least one handle (7).

6. The mobile thermal texture mapping apparatus as claimed in any one of claims 1 to 5 in which one outer surface of the container (6) further comprises wheels (8).

7. The mobile thermal texture mapping apparatus as claimed in any one of claims 1 to 6 in which the self-contained (5) or external power supply (9) supplies 12V DC power.

8. The mobile thermal texture mapping apparatus as claimed in claim 7 in which the 12V DC power is supplied by a self-contained accumulator or an external automobile engine.

9. The mobile thermal texture mapping apparatus as claimed in claim 7 in which the 12V DC power is supplied by an external transformer which performs the transformation and commutation of the power from AC 220V or 110V commercial power, or supplied by an external man-powered generator.

## Patentansprüche

1. Mobile thermische Texturabbildungsvorrichtung, umfassend:
a) einen Infrarot-Scankopf (1),
b) einen richtungssteuernden Schwebetisch (2),
c) eine Scankopf-Richtungssteuerung (3),
d) eine Zentraleinheit (4),
e) eine Energieeinheit, eine unabhängige Energiequelle (5) und/oder eine Schnittstelle für eine externe Energiequelle (9) umfassend, und
f) einen Behälter (6), einen äußeren Behälter und mindestens einen inneren Behälter umfassend,
wobei, wenn die Vorrichtung nicht in Gebrauch ist, der Infrarot-Scankopf (1), der richtungssteuernde Schwebetisch (2), die Scankopf-Richtungssteuerung (3), die Zentraleinheit (4) und die Energieeinheit in dem Behälter (6) untergebracht sind;
und wobei, wenn die Vorrichtung in Gebrauch ist, der Infrarot-Scankopf (1) und der richtungssteuernde Schwebetisch (2) aus dem Behälter (6) entfernt sind und der Infrarot-Scankopf (1) sich auf dem richtungssteuernden Schwebetisch (2) befindet, der von mindestens einer Oberfläche des Behälters (6) getragen wird;
**dadurch gekennzeichnet, dass** die inneren und äußeren Behälter der Vorrichtung weiterhin Gleitschienen und Führungsschlitze umfassen, um vertikale Verlagerung der inneren Behälter relativ zu weiteren inneren Behältern und dem äußeren Behälter zu ermöglichen, und die inneren Behälter durch Befestigungsmittel in Position gesichert werden.

2. Mobile thermische Texturabbildungsvorrichtung nach Anspruch 1, wobei der mindestens eine innere Behälter vertikal relativ zu dem äußeren Behälter verlagert werden kann und an einer erforderlichen vertikalen Verlagerung gesichert werden kann, um eine stabile, tragende Oberfläche für den richtungssteuernden Schwebetisch (2) bereitzustellen.

3. Mobile thermische Texturabbildungsvorrichtung nach Anspruch 2, wobei der Behälter (6) einen äußeren Behälter und zwei innere Behälter umfasst.

4. Mobile thermische Texturabbildungsvorrichtung nach Anspruch 1, wobei die Befestigungsmittel aus Sicherungsstiften oder -blöcken ausgewählt werden.

5. Mobile thermische Texturabbildungsvorrichtung nach einem der Ansprüche 1 bis 4, wobei mindestens eine äußere Oberfläche des Behälters (6) weiterhin mindestens einen Griff (7) umfasst.

6. Mobile thermische Texturabbildungsvorrichtung nach einem der Ansprüche 1 bis 5, wobei eine äußere Oberfläche des Behälters (6) weiterhin Räder (8) umfasst.

7. Mobile thermische Texturabbildungsvorrichtung nach einem der Ansprüche 1 bis 6, wobei die unabhängige (5) oder externe Energiequelle (9) Energie von 12 V Gleichstrom zuführt.

8. Mobile thermische Texturabbildungsvorrichtung nach Anspruch 7, wobei die Gleichstrom-Energie von 12 V von einem unabhängigen Akkumulator oder einem externen Automobilmotor zugeführt wird.

9. Mobile thermische Texturabbildungsvorrichtung nach Anspruch 7, wobei die Gleichstrom-Energie von 12 V von einem externen Transformator, der die Umwandlung und Kommutierung der Energie von Netzstrom von 220 V oder 110 V Wechselstrom durchführt, zugeführt wird oder von einem externen, von Menschen angetriebenen Generator zugeführt wird.

## Revendications

1. Appareil de placage de texture thermique mobile comprenant :
a) une tête de balayage infrarouge (1),
b) une table flottante de commande de sens (2),
c) un contrôleur de sens de tête de balayage (3),
d) une unité centrale (4),
e) un bloc d'alimentation comprenant une alimentation autonome (5) et/ou une interface d'alimentation externe (9), et
f) un logement (6) comprenant un logement externe et au moins un logement interne,
dans lequel quand l'appareil n'est pas utilisé, la tête de balayage infrarouge (1), la table flottante de commande de sens (2), le contrôleur de sens de tête de balayage (3), l'unité centrale (4) et le bloc d'alimentation sont stockées dans le logement (6) ;
et dans lequel quand l'appareil est utilisé, la tête de balayage infrarouge (1) et la table flottante de commande de sens (2) sont sorties du logement (6) et la tête de balayage infrarouge (1) est placée sur la table flottante de commande de sens (2) qui est supportée par au moins une surface du logement (6) ;
**caractérisé en ce que** les logements interne et externe de l'appareil comprennent en outre des rails coulissants et des fentes de guidage pour permettre le déplacement vertical des logements internes par rapport à d'autres logements internes et au logement externe, et lesdits logements internes sont fixés en place par des moyens de fixation.

2. Appareil de placage de texture thermique mobile selon la revendication 1, dans lequel l'au moins un logement interne peut être déplacé verticalement par rapport au logement externe et fixé à un déplacement vertical requis pour servir de surface de support stable à la table flottante de commande de sens (2).

3. Appareil de placage de texture thermique mobile selon la revendication 2, dans lequel le logement (6) comprend un logement externe et deux logements internes.

4. Appareil de placage de texture thermique mobile selon la revendication 1, dans lequel les moyens de fixation sont sélectionnés parmi des broches ou blocs de positionnement.

5. Appareil de placage de texture thermique mobile selon l'une quelconque des revendications 1 à 4, dans lequel au moins une surface externe du logement (6) comprend en outre au moins une poignée (7).

6. Appareil de placage de texture thermique mobile selon l'une quelconque des revendications 1 à 5, dans lequel une surface externe du logement (6) comprend en outre des roues (8).

7. Appareil de placage de texture thermique mobile selon l'une quelconque des revendications 1 à 6, dans lequel l'alimentation autonome (5) ou l'alimentation externe (9) fournit une alimentation C.C. de 12 V.

8. Appareil de placage de texture thermique mobile selon la revendication 7, dans lequel l'alimentation C.C. de 12V est fournie par un accumulateur autonome ou un moteur d'automobile externe.

9. Appareil de placage de texture thermique mobile selon la revendication 7, dans lequel l'alimentation C.C. de 12V est fournie par un transformateur externe qui effectue la transformation et la commutation de l'alimentation depuis une alimentation commerciale C.A. de 220V ou 110V, ou fournie par un générateur actionné manuellement externe.
